(19) **Europäisches Patentamt**

**European Patent Office**

**Office européen des brevets**

(11) **EP 0 676 639 B1**

(12) **EUROPÄISCHE PATENTSCHRIFT**

| | |
|---|---|
| (45) Veröffentlichungstag und Bekanntmachung des Hinweises auf die Patenterteilung:<br>**18.07.2001 Patentblatt 2001/29** | (51) Int Cl.7: **G01N 33/44**, G01N 17/00 |

(21) Anmeldenummer: **95103209.3**

(22) Anmeldetag: **07.03.1995**

(54) **Verfahren und Vorrichtung zur quantitativen Bewertung des Alterungsverhaltens eines polymeren Werkstoffes**

Method and apparatus for quantitatively evaluating ageing behaviour of a polymeric material

Méthode et appareil pour évaluer quantitativement le comportement du vieillissement d'une matière polymère

| | |
|---|---|
| (84) Benannte Vertragsstaaten:<br>**CH DE FR GB LI** | (74) Vertreter:<br>**Stoffregen, Hans-Herbert, Dr. Dipl.-Phys.**<br>**Patentanwalt**<br>**Postfach 21 44**<br>**D-63411 Hanau (DE)** |
| (30) Priorität: **08.03.1994 DE 4407608** | |
| (43) Veröffentlichungstag der Anmeldung:<br>**11.10.1995 Patentblatt 1995/41** | (56) Entgegenhaltungen:<br>**DE-A- 1 904 097    DE-A- 2 502 239**<br>**DE-A- 3 047 370    DE-U- 9 100 816**<br>**GB-A- 2 064 788** |
| (73) Patentinhaber: **Kockott, Dieter Dr.**<br>**63456 Hanau (DE)** | |
| (72) Erfinder: **Kockott, Dieter Dr.**<br>**63456 Hanau (DE)** | |

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die Erfindung bezieht sich auf Verfahren und Vorrichtungen zur quantitativen Bewertung des Alterungsverhaltens eines polymeren Werkstoffes in Abhängigkeit der Temperatur und/oder Wellenlänge der auf den Werkstoff einfallenden Strahlung.

Photochemische Primärprozesse, d.h. die Absorption von Photonen und die Erzeugung angeregter Zustände oder freier Radikale, sind temperaturunabhängig. Dagegen können die nachfolgenden Reaktionsschritte mit den Polymeren oder Additiven temperaturabhängig sein, so daß die beobachtete Alterung der Werkstoffe ebenfalls temperaturabhängig ist. Der Grad der Temperaturabhängigkeit ist vom Werkstoff und der betrachteten Eigenschaftsänderung abhängig.

Um diesen Effekt zu simulieren, werden in Vorrichtungen zur künstlichen Bewitterung von polymeren Werkstoffen die Raumtemperatur und/oder die Schwarztafel/Schwarzstandardtemperatur konstant gehalten. Wegen der Temperaturabhängigkeit der Alterung werden möglichst enge Toleranzen für die Temperatur gefordert. Ein entscheidendes Kriterium für die Güte eines Bewitterungsgerätes ist dabei die Gleichmäßigkeit der Eigenschaftsänderung, z.B. der Verfärbung, auf der gesamten Probenflächen.

Wenn man eine experimentelle Auskunft über die Temperaturabhängigkeit der Alterung einer Probe erlangen will, müssen mehrere Bewitterungen hintereinander bei verschiedenen Probenraumtemperaturen und/oder Schwarztafel/Schwarzstandardtemperaturen durchgeführt werden. Solche Maßnahmen sind erkennbar zeitraubend.

Um diese Nachteile zu vermeiden, ist bereits in der DE 29 48 175 A1 ein Licht- und Wetterechtheits-Prüfgerät beschrieben worden, bei dem entlang der zu überprüfenden Proben ein Temperaturgradient erzeugt wird. Dieser ist vorzugsweise linear und wird durch Wärmeleitung durch z.B. Beheizen oder Kühlen eines Probenträgers erzielt, der z.B. aus diathermem Material besteht.

Das Alterungsverhalten hängt jedoch auch von der spektralen Verteilung der auf die Probe einfallenden Strahlung ab. Dabei wird die durch die Sonnenstrahlung verursachte Alterung von organischen Stoffen von der effektiv wirksamen Bestrahlungsstärke $E_{eff}$ bestimmt. Materialeigenschaftsänderungen sind dabei in erster Näherung proportional der effektiven Bestrahlung (Dosis)

$$H_{eff} = \int E_{eff}\, dt$$

Nach dem Stand der Technik wird die Strahlung einer polychromatischen Strahlenquelle, z.B. eines Xe-Strahlers durch ein Prisma oder Gitter in ihre spektralen Anteile zerlegt und auf eine ebene Probe gerichtet. Dadurch werden die verschiedenen Teile der Probe mit Strahlung unterschiedlicher Wellenlängen bestrahlt. Die Eigenschaftsänderungen an verschiedenen Stellen der Probe können eindeutig der Wellenlänge der aufgetroffenen Strahlung zugeordnet werden. Dem Stand der Technik ist jedoch der Nachteil immanent, daß lange Bestrahlungsdauern erforderlich sind, da die spektrale Bestrahlungsstärke auf der Probe selbst relativ gering ist.

Bei einem Licht- und Wetterechtheitsprüfgerät nach der DE 25 02 239 B2 wird ein Werkstoff mittels z. B. eines Xe-Strahlers bestrahlt, wobei dessen IR-Anteil selektiv mittels eines Spiegels ausgefiltert wird.

Um die spektrale Lichtbeständigkeit z.B. einer Kunststoffprobe zu bestimmen, werden nach der DE-AS 1 904 097 zwischen Probe und Strahlungsquelle Kantenfilter angeordnet.

Um eine Probe im erforderlichen Umfang zu erwärmen, werden nach der DE 28 16 548 A1 mehrere IR-Strahler oberhalb der Probe angeordnet.

Nach der EP 0 487 202 A1 bzw. US 4,874,952 werden in Licht- bzw. Wetterechtheitsprüfgeräten Linienstrahler wie Metall-Halegonid-Strahler benutzt.

Dem DE 91 00 816 U1 ist eine Bestrahlungsvorrichtung zur Sonnensimulation mit einer Halogenidlampe für eine Strahlung im UV- und VIS-Bereich und eine Halogenglühlampe für eine Strahlung im IR-Bereich zu entnehmen.

Weitere Vorrichtungen und Verfahren zur Alterungsbestimmung von polymeren Werkstoffen sind z.B. der DE 25 43 876 A1, DE 28 16 548 A1, EP 0 320 209 A2, EP 0 487 202 A1, EP 0 550 970 A2, DE-AS 19 04 097, DE-OS 20 14 288, US 3,797,918, US 4,544,995, US 4,760,748, US 5,226,318, US 4,874,952, DE 32 21 392 A1, DE 25 02 239 B2, DE 34 43 604 A1, DE 30 47 370 C2, DE 40 02 985 A1, DE 37 26 803 C1, DE 34 30 426 C1, EP 0 289 436 A2, DE 37 20 117 A1, DE 35 04 793 C2 oder EP 0 302 986 A2 zu entnehmen.

Der vorliegenden Erfindung liegt u.a. das Problem zugrunde, ein Verfahren bzw. Vorrichtungen zur quantitativen Bewertung des Alterungsverhaltens eines polymeren Werkstoffes in Abhängigkeit der Temperatur und/oder Wellenlänge der auf den Werkstoff einfallenden Strahlung so weiterzubilden, daß Verhältnisse vorliegen, die den natürlichen entsprechen, wobei im Vergleich zur Wärmeleitung auf alternative Weise ein Temperaturgradient in dem Werkstoff erzeugt werden soll.

Auch soll die Möglichkeit gegeben werden, in kurzer Zeit Aussagen über die spektrale Empfindlichkeit des Werkstoffs und die damit verbundenen Alterungserscheinungen treffen zu können.

Vorrichtungsgemäß wird das Problem unter anderem dadurch gelöst, daß der Vorrichtung zumindest ein Infrarot(IR)-Strahler zur Erzeugung des Temperatur-Gradienten entlang einer Richtung des Werkstoffes zugeordnet ist. Insbesondere wird das Problem jedoch dadurch gelöst, daß die Vorrichtung zumindest einen Infrarot(IR)-Strahler zur Erzeugung des Temperatur-Gradienten entlang einer Richtung des Werkstoffs sowie zumindest einen Gasentladungsstrahler aufweist, wobei unterschiedliche Bereiche des Werkstoffs mit Licht unterschiedlicher Wellenlängen bestrahlbar sind.

Dabei kann vorgesehen sein, daß ein langgestreckter IR-Strahler im Bereich eines Randes des Werkstoffes und senkrecht zur Richtung oder mehrere IR-Strahler vorzugsweise unterschiedlicher Leistungen entlang der Richtung angeordnet sind, um so ein treppenförmiges Temperatur-Profil im Werkstoff zu erzeugen.

Um die spektrale Empfindlichkeit des Werkstoffes reproduzierbar und in kurzer Zeit bestimmen zu können, ist vorgesehen, daß der auf den Werkstoff ausgerichtete Gasentladungsstrahler ein Linienstrahler ist, dessen Strahlungsfluß in wenigen Spektrallinien konzentriert ist.

Vorzugsweise kann ein Gasentladungsstrahler in Form eines Quecksilber(Hg)-Hochdruckstrahlers mit Zusätzen wie Indium- oder Thallium-Verbindungen zum Einsatz gelangen, die das Linienspektrum im Ultraviolett(UV)-Bereich auffüllen. Auch kann der Gasentladungsstrahler ein Metall-Halogenid-Strahler oder ein Xe-Strahler mit entsprechenden Filtervorsätzen sein.

Zusätzlich besteht die Möglichkeit, daß zwischen dem Gasentladungsstrahler und dem Werkstoff Prismen oder Gitter angeordnet werden, um die von dem Strahler emittierten Spektrallinien aufzufächern.

Aufgrund der erfindungsgemäßen Lehre ist die spektrale Bestrahlungsstärke der Spektrallinien wesentlich größer im Vergleich zu der spektralen Bestrahlungsstärke einzelner Wellenlängen einer polychromatischen Strahlenquelle wie Xenon(Xe)-Strahler ohne Filtervorsätze, so daß infolgedessen mit kürzeren Bestrahlungszeiten gearbeitet werden kann.

Alternativ kann der Werkstoff ohne Zwischenschaltung von Prismen oder Gittern bestrahlt werden. In diesem Fall müssen jedoch vor dem Werkstoff mehrere schmalbandige Interferenzfilter nebeneinander angeordnet werden, deren $\lambda_{max}$ mit der Wellenlänge der Einzelspektrallinien übereinstimmen. Der hinter dem Interferenzfilter liegende Teil der Probe wird sodann nur von monochromatischer Strahlung der Wellenlänge $\lambda_{max}$ bestrahlt.

Verfahrensmäßig zeichnet sich die Erfindung dadurch aus, daß der Werkstoff mit Infrarot-Strahlung zur Erzeugung des Temperatur-Gradienten bestrahlt wird. Auch ist vorgesehen, daß der Werkstoff einerseits mit Infrarot-Strahlung zur Erzeugung des Temperatur-Gradienten und andererseits örtlich unterschiedliche Bereiche des Werkstoffs mit Licht unterschiedlicher Wellenlänge bestrahlt wird bzw. werden.

Durch die Verwendung von Infrarot-Strahlung werden die natürlichen Verhältnisse, denen eine Probe bei Bestrahlung mit Sonnenlicht ausgesetzt ist, realistisch nachgebildet, eine Möglichkeit, die bei Alterungsverfahren nach dem Prinzip der Wärmeleitung zumindest bezüglich des Temperaturverlaufs innerhalb der Probe nicht gegeben ist.

Daher zeichnet sich die Erfindung auch dadurch aus, daß zur gezielten Erzeugung eines nichtlinearen Temperaturverlaufs senkrecht zur Oberfläche des Werkstoffs dieser mit Infrarot-Strahlung beaufschlagt wird.

Insbesondere zeichnet sich die Erfindung auch dadurch aus, daß der Gasentladungsstrahler vorzugsweise im Brennpunkt eines im Schnitt parabolförmigen bzw. einen Teil einer Ellipse darstellenden Reflektors angeordnet wird, daß von dem Gasentladungsstrahler und dem Reflektor kommende Strahlung auf einen selektiv reflektierenden Filter fällt und daß von dem Filter reflektierte Strahlung gegebenenfalls unter Zwischenschaltung von zumindest einem weiteren Filter auf die Probe abgestrahlt wird, wobei die Strahlung derart selektiert wird, daß diese der kurzwelligen Kante der Spektralverteilung des Sonnenlichts entspricht.

Weitere Einzelheiten, Vorteile und Merkmale der Erfindung ergeben sich nicht nur aus den Ansprüchen, den diesen zu entnehmenden Merkmalen -für sich und/oder in Kombination-, sondern auch aus der nachfolgenden Beschreibung von der Zeichnung zu entnehmenden bevorzugten Ausführungsbeispielen.

Es zeigen:

Fig. 1 eine Prinzipdarstellung eines Prüfraums zur quantitativen Bewertung des Alterungsverhaltens eines polymeren Werkstoffes in Abhängigkeit der Temperatur und/oder der Wellenlänge von auf den Werkstoff einfallender Strahlung,

Fig. 2 eine zweite Ausführungsform eines entsprechenden Prüfraums,

Fig. 3 ein Temperaturverlauf an der Oberfläche des Werkstoffs in dem Prüfraum,

Fig. 4 eine Prinzipdarstellung einer Prüfungsanordnung und

Fig. 5 eine weitere Ausführungsform zur Erzeugung eines Temperaturprofils auf einer Probenfläche.

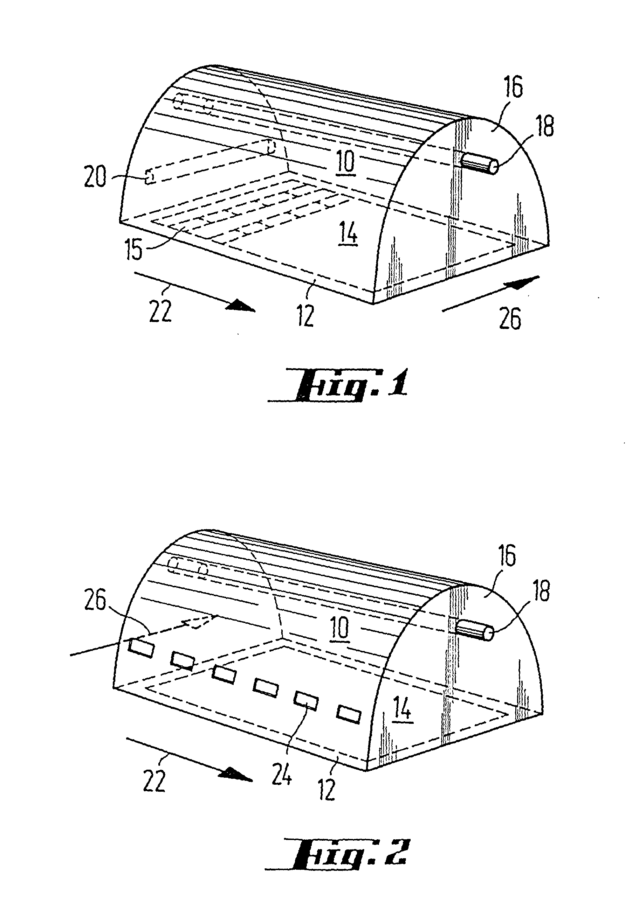

In den Fig. 1 und 2 ist rein prinzipiell ein Prüfraum (10) dargestellt, auf dessen Boden eine Probe (14) eines polymeren Werkstoffes angeordnet wird, um dessen Alterungsverhalten in Abhängigkeit von Temperatur und Wellenlänge von auf den Werkstoff einfallender Strahlung quantitativ zu bewerten. Der Prüfraum (10) ist von einem Reflektor (16) umgeben.

Bei dem Reflektor (16) kann es sich um einen Parabolreflektor handeln, in dessen Brennbereich ein sich entlang der Probe (14) erstreckender Gasentladungsstrahler (18) angeordnet ist. Bei dem Gasentladungsstrahler (18) handelt es sich um einen Linienstrahler, dessen Strahlungsfluß in wenigen Spektrallinien konzentriert ist. Dabei können aus der Praxis bekannte Metall-Halogenid-Strahler mit Zusätzen wie Indium- und Thallium-Verbindungen benutzt werden. Sogar Xe-Strahler mit entsprechenden Filtervorsätzen könnten verwendet werden.

Zwischen dem Strahler (18) und der Probe (14) sind des weiteren Interferenzfilter (15) angeordnet.

Nach dem Ausführungsbeispiel der Fig. 1 erstreckt sich in einer Richtung (26) senkrecht zum Gasentladungsstrahler (18), also entlang einer Stirnseite der Probe (14) ein Infrarot(IR)-Strahler (20), durch den die Probe (14) derart aufgewärmt wird, daß die Temperatur mit zunehmender Entfernung von dem Strahler (20) abnimmt. Es wird folglich ein Temperatur-Gradient längs der Probe (14) erzeugt. Aufgrund der geringen Quantenenergie der Infrarot-Strahlung werden jedoch keine photochemischen Prozesse in dem polymeren Werkstoff ausgelöst. Dennoch wird die Probe (14) infolge der Absorption an der Oberfläche und im Inneren in gleicher Weise erwärmt wie ein Werkstoff, der Sonnenstrahlung ausgesetzt ist.

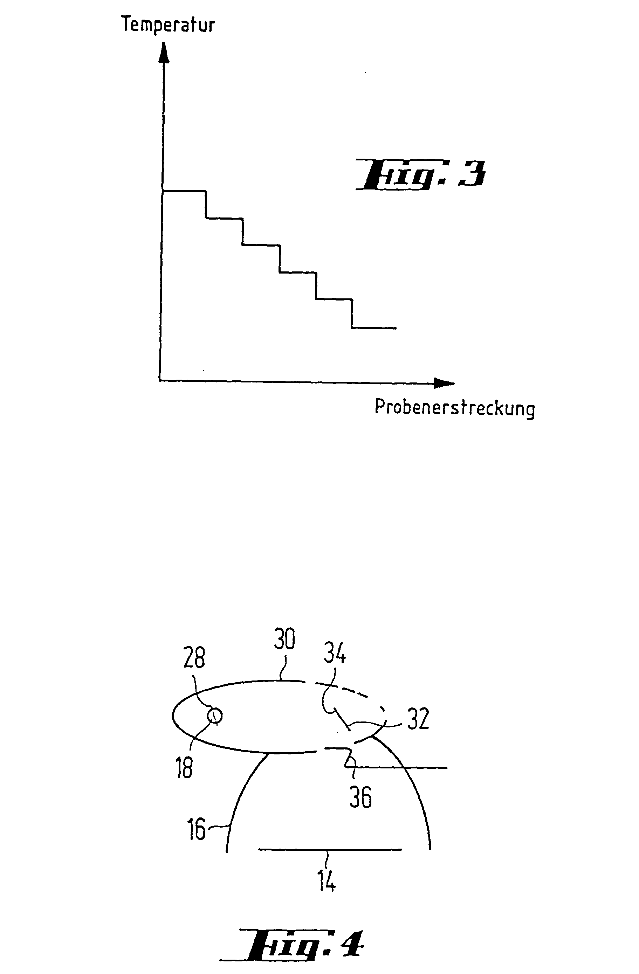

Um in der Probe (14) ein treppenförmiges Temperatur-Profil zu erzeugen, wie es beispielhaft in der Fig. 3 dargestellt ist, können entlang der Probe (14), also entlang einer Richtung (22), die parallel zur Längsachse des Linienstrahlers (18) verläuft, mehrere Infrarot-Strahler (24) angeordnet werden, die unterschiedliche Strahlungsflüsse aufweisen. Auf diese Weise kann über eine größere flächige Erstreckung, in der eine einheitliche Temperatur vorliegt, eine bessere quantitative Bewertung der Probe wie z.B. durch Farbmessung vorgenommen werden.

Um gleichzeitig die spektrale Empfindlichkeit der Probe (14) und die damit verbundenen Alterungserscheinungen zu bestimmen, ohne daß es langer Bestrahlungszeiten bedarf, können zwischen der Probe (14) und dem Linienstrahler (18) Prismen oder Gitter angeordnet werden, um die Spektrallinien aufzufächern. Dabei erfolgt eine Auffächerung derart, daß die Richtung (26), entlang der die Probe (14) mit Strahlungen unterschiedlicher Wellenlänge bestrahlt wird, senkrecht zu der Richtung (22) verläuft, entlang der der Temperatur-Gradient zuvor beschriebener Art erzeugt wird.

Alternativ kann die Probe (14) ohne Zwischenschaltung von Prismen oder Gittern bestrahlt werden. Vor der Probe (14) müssen dann mehrere schmalbandige Interferenzfilter (15) nebeneinander angeordnet werden, deren $\lambda_{max}$ mit der Wellenlänge der Einzelspektrallinien des Linienstrahlers (18) übereinstimmen. Der hinter dem Interferenzfilter liegende Teil der Probe wird sodann nur von monochromatischer Strahlung der Wellenlänge $\lambda_{max}$ bestrahlt.

Die Messung des Temperaturgradienten bzw. der Temperatur der einzelnen Bestrahlungsfelder erfolgt in bekannter Weise, z.B. durch abgedeckte oder eingearbeitete Thermoelemente oder Thermowiderstände oder berührungslos durch Messung der vom Material emittierten IR-Strahlung.

Xe-Strahler und -in geringem Maße- Metall-Halogenid-Strahler erzeugen neben der gewünschten UV- und sichtbaren (VIS)Strahlung einen erheblichen Anteil an IR-Strahlung, der bei Untersuchungen im niedrigen Temperaturbereich störend ist und durch IR-Filter ausgefiltert werden muß. Aus diesem Grunde sind auch Fluoreszenzlampen, die vornehmlich im UV emittieren, vorteilhaft verwendbar. Eine neue kostengünstige IR- bzw. VIS-Filterung im Falle von Xe- und/oder Metall-Halogenid-Strahlern (18) könnte gemäß Fig. 4 aufgebaut sein.

Der langgestreckte Strahler (18) befindet sich in einem Brennpunkt (28) eines im Schnitt parabolförmigen bzw. eines Teils einer Ellipse darstellenden Reflektors (30). In deren zweitem Brennpunkt (32) ist ein Spiegel (34) angeordnet, der nur UV (+ VIS) reflektiert, die IR-Strahlung aber hindurchläßt. Die reflektierte Strahlung wird zusätzlich durch ein oder mehrere Filter (36) angepaßt. Die Strahlung trifft dann entsprechend der in Fig. 1 dargestellten Weise auf die Probe (14). Die Strahlung selbst entspricht der kurzwelligen Kante der Spektralverteilung des Sonnenlichts.

Wenn man Spiegel (34) und Filter (36) so wählt, daß nur die photochemisch wirksame Strahlung (z.B. $\lambda \le 450$ nm) auf die Probe (14) auftrifft, dann kann man auf der Probe (14) erheblich höhere Bestrahlungsstärken (bis Faktor 20) erzeugen, mit der Folge entsprechend kürzerer Prüfzeiten, ohne daß sich die Probe (14) unzulässig erwärmt. Die gewünschte Temperatur auf der Probe (14) wird dann erfindungsgemäß durch die zusätzliche IR-Strahlung gezielt und reproduzierbar erzeugt.

Im F&E-Bereich ist bei photooxidativen Abbauprozessen auch die aufgenommene Menge $O_2$ und die Art und Menge der gasförmigen Abbauprodukte von Interesse. Für diese Aufgabe können die Proben (14) in Quarz-Küvetten bestrahlt werden, indem ein Gasgemisch mit unterschiedlichen $O_2$-Partialdrucken eingeschlossen ist oder im flow system durchströmt.

Aufgrund der Kenntnis der spektralen Empfindlichkeit und der Temperaturabhängigkeit der Alterung kann man aus dem Alterungsverhalten eines Werkstoffes an einem Ort der Erde das Alterungsverhalten an anderen Orten der Erde mit anderen klimatischen Bedingungen (Strahlung und Temperatur) vorhersagen.

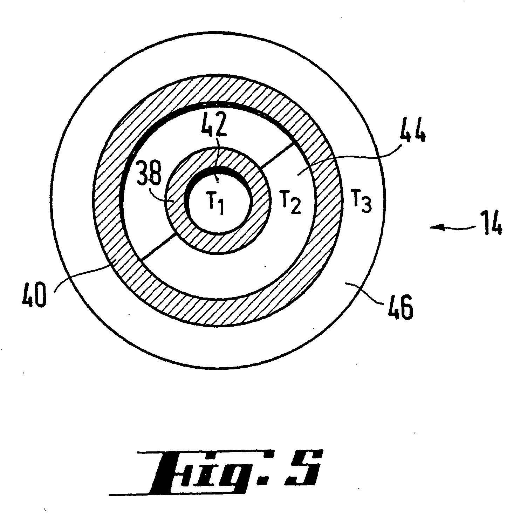

Um eine insbesondere visuelle Überprüfung einer Probenoberfläche zu ermöglichen, von der Bereiche mit unterschiedlichen Temperaturen beaufschlagt werden sollen, kann ein axial-symmetrisches Temperaturprofil gemäß Fig. 5 dadurch erzeugt werden, daß von einer axial-symmetrisch Infrarotstrahlung abstrahlenden Lichtquelle unterhalb dieser verlaufende Bereiche einer Probe (14) mit konzentrisch zueinander verlaufenden ringförmigen Abdeckungen (38), (40) abgeschattet wie abgedeckt werden. Hierdurch ergibt sich der Vorteil, daß die zwischen den Abdeckungen (38), (40) vorhandenen Bereiche (42), (44), (46) unterschiedlichen Temperaturen ausgesetzt werden, so daß deren optische Veränderungen gegenüber der Ursprungsoberfläche dann visuell erfaßt werden können, wenn die Abdeckungen (38), (40) entfernt werden.

Mit anderen Worten kann auf einfache Weise nach Ende einer Exposition die Farbänderung einer Probe (14) gegenüber unbestrahlten Bereichen der Probe visuell als Funktion der Temperaturen $T_1$, $T_2$, $T_3$ ... bestimmt werden.

**Patentansprüche**

1. Vorrichtung zur quantitativen Bewertung des Alterungsverhaltens eines polymeren Werkstoffes (14) in Abhängigkeit von Temperatur und/oder Wellenlänge der auf den Werkstoff einfallenden Strahlung umfassend einen den Werkstoff vorzugsweise in Form einer flächigen Probe aufnehmenden Prüfraum (10), einen auf den Werkstoff ausgerichteten Gasentladungsstrahler (18) sowie eine Einrichtung (20, 24, 26) zur Erzeugung eines Temperatur-Gradienten in dem Werkstoff,
**dadurch gekennzeichnet**,
daß die Vorrichtung zumindest einen Infrarot(IR)-Strahler (20, 24) zur Erzeugung des Temperatur-Gradienten entlang einer Richtung (22) des Werkstoffs (14) aufweist.

2. Vorrichtung nach Anspruch 1,
**dadurch gekennzeichnet,**
daß die Vorrichtung zumindest einen Linienstrahler als Gasentladungsstrahler (18) aufweist, wobei unterschiedliche Bereiche des Werkstoffs (14) mit Licht unterschiedlicher Wellenlängen bestrahlbar sind.

3. Vorrichtung nach Anspruch 1 oder 2,
**dadurch gekennzeichnet**,
daß zumindest ein langgesteckter IR-Strahler (20) im Bereich eines Randes des Werkstoffes (14) und senkrecht zur Richtung (22) oder mehrere IR-Strahler (24) unterschiedlicher Leistungen entlang der Richtung (22) angeordnet sind.

4. Vorrichtung nach Anspruch 1 oder 2,
**dadurch gekennzeichnet,**
daß der Gasentladungsstrahler (18) ein Quecksilber(Hg)-Hochdruckstrahler mit Zusätzen wie Indium- und Thallium-Verbindungen ist, die das Linienspektrum im Ultraviolett(UV)-Bereich auffüllen, oder ein Metall-Halogenid-Strahler oder ein Xe-Strahler mit geeigneter Filterung ist, wobei gegebenenfalls zwischen dem Gasentladungsstrahler (18) und dem Werkstoff (14) Prismen oder Gitter zur geometrischen Auffächerung der Spektrallinien und/oder zwischen dem Gasentladungsstrahler und dem Werkstoff mehrere Interferenzfilter angeordnet sind.

5. Vorrichtung nach zumindest einem der vorhergehenden Ansprüche,
**dadurch gekennzeichnet**,
daß sich die Strahlung unterschiedlicher Wellenlängen entlang einer Richtung (26) erstreckt, die senkrecht zu der Richtung (22) verläuft, entlang der die IR-Strahler (24) ein treppenförmiges Temperatur-Profil erzeugen.

6. Verfahren zur quantitativen Bewertung des Alterungsverhaltens eines polymeren Werkstoffes (14) in Abhängigkeit von Temperatur und/oder Wellenlänge der auf den Werkstoff einfallenden Strahlung, wobei entlang des Werkstoffes ein Temperatur-Gradient erzeugt wird,
**dadurch gekennzeichnet,**
daß der Werkstoff (14) mit Infrarot-Strahlung zur Erzeugung des Temperatur-Gradienten bestrahlt wird.

7. Verfahren nach Anspruch 6,
**dadurch gekennzeichnet,**
daß örtlich unterschiedliche Bereiche des Werkstoffs (14) mit Licht unterschiedlicher Wellenlänge bestrahlt wird bzw. werden.

8. Verfahren nach Anspruch 6 oder 7,
**dadurch gekennzeichnet,**
daß in dem Werkstoff (14) ein treppenförmiges Temperatur-Profil erzeugt wird.

9. Verfahren nach zumindest einem der Ansprüche 6 - 8,
**dadurch gekennzeichnet,**
daß in dem Werkstoff (14) senkrecht zu einer Richtung (26), in der seine spektrale Empfindlichkeit ermittelt wird, der Temperatur-Gradient erzeugt wird.

10. Verfahren nach zumindest einem der Ansprüche 6 - 9,
**dadurch gekennzeichnet**,
daß zur gezielten Erzeugung eines nichtlinearen Temperaturverlaufs senkrecht zur Oberfläche des Werkstoffs (14) dieser mit Infrarot-Strahlung beaufschlagt wird.

11. Verfahren nach zumindest einem der Ansprüche 6 - 10,
**dadurch gekennzeichnet,**
daß der Gasentladungsstrahler vorzugsweise im Brennpunkt eines im Schnitt parabolförmigen oder Teil einer Ellipse darstellenden Reflektors (30) angeordnet wird, daß von dem Gasentladungsstrahler (18) und dem Reflektor kommende Strahlung auf einen selektiv reflektierenden Filter (34) fällt und daß von dem Filter reflektierte Strahlung gegebenenfalls unter Zwischenschaltung von zumindest einem weiteren Filter (36) auf die Probe (14) abgestrahlt wird, wobei die Strahlung derart selektiert wird, daß diese der kurzwelligen Kante der Spektralverteilung des Sonnenlichtes entspricht.

12. Verfahren nach zumindest Anspruch 6,
**dadurch gekennzeichnet,**
daß eine Infrarot-Strahlungsquelle benutzt wird, deren Strahlung axial-symmetrisch auf die Probe (14) angestrahlt wird, wobei die Probe mittels vorzugsweise konzentrisch verlaufenden ringförmigen Abdeckungen (38, 40) bereichsweise abgeschattet wie abgedeckt wird.

## Claims

1. Device for quantitatively evaluating the ageing behaviour of a polymeric material (14) as a function of temperature and/or wavelength of the radiation incident on the material, comprising a test chamber (10) receiving the material, preferably in the form of a flat sample, a gas discharge radiator (18) oriented toward the material and a device (20, 24, 26) for producing a temperature gradient in the material, characterised in that the device has at least one infrared (IR) radiator (20, 24) for producing the temperature gradient along a direction (22) of the material (14).

2. Device according to claim 1, characterised in that the device has at least one line radiator as gas discharge radiator (18), wherein different regions of the material (14) can be irradiated with light of different wavelengths.

3. Device according to claim 1 or 2, characterised in that at least one elongated IR radiator (20) is arranged in the region of an edge of the material (14) and perpendicular to the direction (22) or a plurality of IR radiators (24) of different power are arranged along the direction (22).

4. Device according to claim 1 or 2, characterised in that the gas discharge radiator (18) is a mercury (Hg) high pressure radiator with additives such as indium and thallium compounds which fill the line spectrum in the ultraviolet (UV) range, or a metal halide radiator or an Xe radiator with suitable filtration, prisms or grids optionally being arranged between the gas discharge radiator (18) and the material (14) for geometric fanning of the spectral lines and/or a plurality of interference filters being arranged between the gas discharge radiator and the material.

5. Device according to at least one of the preceding claims, characterised in that the radiation of different wavelengths extends along a direction (26) which runs perpendicular to the direction (22) along which the IR radiators (24) produce a stepped temperature profile.

6. Process for quantitatively evaluating the ageing behaviour of a polymeric material (14) as a function of temperature and/or wavelength of the radiation incident on the material, a temperature gradient being produced along the material, characterised in that the material (14) is irradiated with infrared radiation to produce the temperature gradient.

7. Process according to claim 6, characterised in that different regions of the material (14) is or are irradiated by light of different wavelength.

8. Process according to claim 6 or 7, characterised in that a stepped temperature profile is produced in the material (14).

9. Process according to at least one of claims 6 to 8, characterised in that the temperature gradient is produced in the material (14) perpendicular to a direction (26) in which its spectral sensitivity is determined.

10. Process according to at least one of claims 6 to 9, characterised in that in order intentionally to achieve a nonlinear temperature trend perpendicular to the surface of the material (14) the material is subjected to infrared radiation.

11. Process according to at least one of claims 6 to 10, characterised in that the gas discharge radiator is preferably arranged in the focal point of a reflector (30) having a parabolic shape or part of an ellipse in cross-section, in that radiation issuing from the gas discharge radiator (18) and the reflector strikes a selectively reflecting filter (34) and in that radiation reflected by the filter is radiated onto the sample (14), optionally with interposition of at least one additional filter (36), the radiation being selected in such a way that it corresponds to the short-wave edge of the spectral distribution of sunlight.

12. Process according to at least claim 6, characterised in that an infrared radiation source is used, the radiation of which is radiated symmetrically about the axis onto the sample (14), the sample being shaded and covered in certain regions by means of preferably concentrically extending annular coverings (38, 40).

**Revendications**

1. Dispositif d'évaluation quantitative du comportement de vieillissement d'un matériau polymère (14) en fonction de la température et/ou de la longueur d'onde du rayonnement incident sur le matériau, comprenant un espace test (10) incluant le matériau de préférence sous la forme d'un échantillon plat, un appareil d'irradiation avec décharge de gaz (18) disposée sur le matériau ainsi qu'un dispositif (20, 24, 26) pour produire un gradient de température dans le matériau,
caractérisé en ce que
le dispositif présente au moins un appareil d'irradiation infrarouge (IR)(20, 24) pour obtenir un gradient de température le long d'une direction (22) du matériau (14).

2. Dispositif selon la revendication 1,
caractérisé en ce que
le dispositif présente au moins un appareil d'irradiation linéaire sous la forme d'un appareil à décharge de gaz (18), des zones différentes du matériau (14) pouvant être irradiées avec une lumière de différentes longueurs d'onde.

3. Dispositif selon la revendication 1 ou 2,
caractérisé en ce qu'
au moins un appareil d'irradiation IR longiligne (20) est disposé dans la zone du bord d'un matériau (14) et perpendiculairement à la direction (22), ou plusieurs appareils d'irradiation IR (24) de puissances différentes le long de la direction (22).

4. Dispositif selon la revendication 1 ou 2,
caractérisé en ce que
l'appareil d'irradiation avec décharge de gaz (18) est un appareil d'irradiation haute pression au mercure (Hg) avec des additifs comme des composés d'indium et de thallium, qui remplissent le spectre linéaire dans la zone de l'ultraviolet (UV), ou un appareil d'irradiation à halogénure métallique ou un appareil d'irradiation au xénon avec filtrage approprié, en disposant le cas échéant entre l'appareil d'irradiation à décharge de gaz (18) et le matériau (14) des prismes ou des grilles pour la décomposition géométrique des lignes spectrales, et/ou plusieurs filtres d'interférence entre l'appareil d'irradiation à décharge de gaz et le matériau.

5. Dispositif selon au moins l'une des revendications précédentes,
caractérisé en ce que
le rayonnement de longueurs d'onde différentes s'étend le long d'une direction (26), qui est perpendiculaire à la direction (22) le long de laquelle les appareils d'irradiation IR (24) produisent un profil de température en escalier.

6. Procédé de détermination quantitatif du comportement de vieillissement d'un matériau polymère (14) en fonction de la température et/ou de la longueur d'onde du rayonnement incident sur le matériau, dans lequel le long du matériau on produit un gradient de température,
caractérisé en ce que
le matériau (14) est irradié par un rayonnement infrarouge pour produire le gradient de température.

7. Procédé selon la revendication 6,
caractérisé en ce qu'
on irradie localement différentes zones du matériau (14) avec une lumière de longueurs d'ondes différentes.

8. Procédé selon la revendication 6 ou 7,
caractérisé en ce qu'
on produit dans le matériau (14) un profil de température en escalier.

9. Procédé selon au moins l'une quelconque des revendications 6 à 8,
caractérisé en ce qu'
on produit le gradient de température dans le matériau (14) perpendiculairement à une direction (26) dans laquelle la sensibilité spectrale est déterminée.

10. Procédé selon au moins l'une quelconque des revendications 6 à 9,
caractérisé en ce que
pour la production ciblée d'une propagation de température non linéaire, on bombarde perpendiculairement la surface du matériau (14) avec un rayonnement infrarouge.

11. Procédé selon au moins l'une quelconque des revendications 6 à 10,
caractérisé en ce qu'
on dispose l'appareil d'irradiation avec décharge de gaz de préférence dans le foyer d'un réflecteur (30) en forme de parabole ou représentant une partie d'ellipse, le rayonnement provenant de l'appareil d'irradiation avec décharge de gaz (18) et du réflecteur tombe sur un filtre à réflexion sélective (34), et le rayonnement réfléchi par le filtre est renvoyé sur l'échantillon (14) le cas échéant avec interposition d'au moins un autre filtre (36), le rayonnement étant choisi de manière qu'il corresponde à la limite de longueur d'onde courte de la répartition spectrale de la lumière solaire.

12. Procédé selon au moins la revendication 6,
caractérisé en ce qu'
on utilise une source de rayonnement infrarouge dont le rayonnement est projeté avec une symétrie axiale sur l'échantillon (14), l'échantillon étant ombré, comme par exemple recouvert, au moyen de revêtements annulaires, de préférence concentriques (38, 40) dans certaines zones.

**Fig. 1**

**Fig. 2**

Temperatur

Fig. 3

Probenerstreckung

Fig. 4

Fig. 5